# EUROPEAN PATENT APPLICATION

(11) **EP 1 961 390 A1**
(43) Date of publication of application: **27.08.2008**
(21) Application number: 05816870.9
(22) Date of filing: 15.12.2005
(51) Int. Cl.: A61B 17/3211, A61B 19/02

(54) **BLADE STOWAGE CASE**

(71) Applicant: ACP Japan Co. Ltd., Tokyo 113-0033 (JP); Nakamura, Shoichi, Higashichikuma-gun Nagano 399-7502 (JP)
(72) Inventor: NAKAMURA, Shoichi, Higashichikuma-gun, Nagano 399-7502 (JP)
(74) Representative: Gill, David Alan
(86) International application number: PCT/JP2005/023036
(87) International publication number: WO 2007/069324

(57) **Abstract**

The invention provides a blade storage case which enables a blade to be attached to a holder body without touching the blade with the blade accommodated in the case, is safe and enables bacteria and the like to be prevented from adhering.

The holder body is provided at its front end portion with an attaching finger to attach the blade, and in the blade is formed a fit hole adapted to an engagement portion such as a slit or the like formed in the attaching finger. The storage case that accommodates the blade is provided with an upper support face and a lower support face to sandwich upper and lower surfaces of the plate-shaped blade therebetween to store. As the upper and lower support faces, for example, the case is divided into two parts, and the upper support face is formed in the upper case while the lower support face is formed in the lower case. Alternately, an internal lid is provided to form the upper support face. In the box-shaped case body, an insertion guide hole to guide the attaching finger along the lower surface of the internal blade from the outside of the case and a blade fixing piece that positions and holds the blade are disposed in at least one of the upper and lower support faces.

## Description

The present invention relates to a storage case that accommodates a surgical knife blade (hereinafter, referred to as a "blade" as appropriate) for surgical operations, and more particularly, to a storage case of a blade to attach and detach the blade as an attachment to/from a front end of a handle portion of a holder or the like.

Conventionally, as a surgical knife, there have been known two types that a handle portion to be grasped when used and a blade edge portion at the front end of the handle portion are formed integrally, and that a blade and a holder are formed separately while the blade is attached to the front end of the handle when used. The latter type of knife such that the handle and blade are manufactured and supplied separately and incorporated when used is widely used. When such a knife is a knife for work, the blade is exchanged with a new blade according to the wearing state of the blade edge. Meanwhile, when such a knife is a surgical knife, the knife is used as disposal equipment while a used knife blade is discarded and exchanged with a new blade. Conventionally, in the case where a blade is attached or detached to/from the handle portion to be grasped when used, a user engages the blade in an engagement portion such as a slit provided at the front end portion of the handle to attach or detaches the blade therefrom to exchange, by hand and fingers, or using a device such as a forceps or the like.

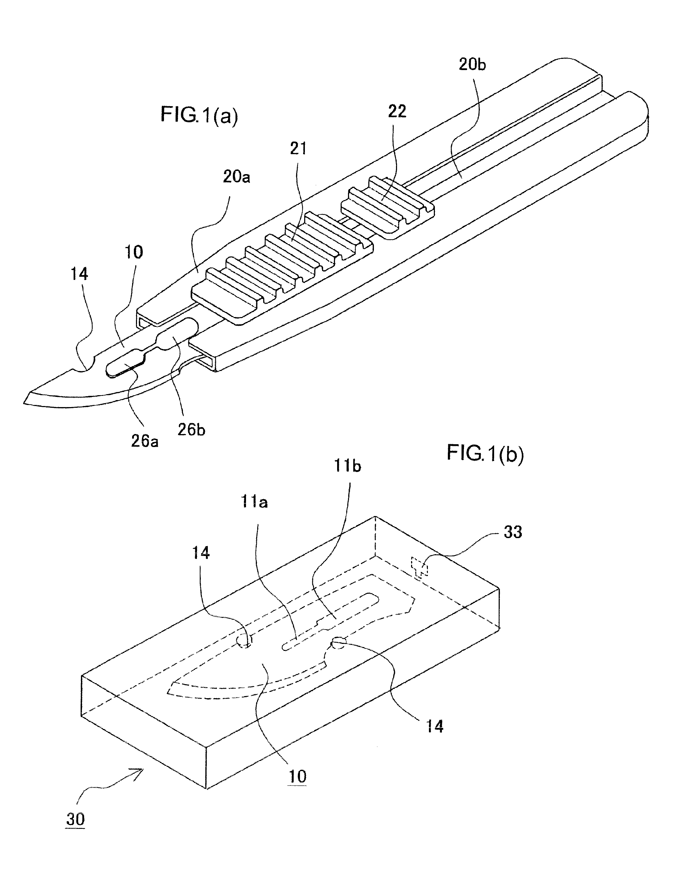

However, since blades for dedicated purposes such as the surgical knife and the like are formed particularly acutely, there is a risk of injury such as hurt and the like when the surgical knife is attached or detached, and it is required to attach and detach the surgical knife safely and easily. Therefore, in the case of the surgical knife, for example, Patent Document 1 (JP 2003-504145) proposes in FIG.1 a structure where a case-shaped guard is provided, a blade is slidably attached to the case-shaped guard, the guard incorporating the blade is fitted and attached to a handle, and the internal blade is caused to protrude outside the guard with an operational slider provided on the handle side and used. Accordingly, the method is proposed where the guard holder with the internal blade is attached and fixed to the handle to be used, and the guard holder is removed from the handle and discarded after being used. The publication describes sterilizing the guard holder to distribute, and thereby preventing infection when the guard holder is attached to the handle and used.

As described above, when a blade is attached as an attachment to a holder in accordance with the intended use or detached to be discarded, it is danger for a user to directly hold the blade using fingers and the like to attach or detach, and it is pointed out also in Patent Document 1 that exchanging an acute blade such as a surgical knife or the like in emergency results in a risk of unintentional damage. Therefore, Patent Document 1 discloses the method and structure where an attachment (guard holder as described previously) with an internal blade is attached to a handler detachably. Such a type that the attachment with the internal blade is attached and detached is expensive because the guard holder is exchanged, and has problems that the attachment rattles when attached to the handle and that the operation for loading or unloading the blade is not performed smoothly unless the attachment is manufactured precisely.

Further, it is not possible to adapt such an attachment to a handle to attach using fingers which are already commercially available and used. Furthermore, when bacteria adhere to the blade or the holder protecting the blade in a medical operation and the like, there is a risk of infection, and the problem arises that the holder with the internal blade needs sterilization packing to be distributed. Thus, the method of Patent Document 1 has defects that the manufacturing cost is high and that the distribution cost is also high because consideration must be given to distribution to prevent bacteria from adhering and the attachment from being deformed by impact and the like. Therefore, it is an object of the invention to provide a blade storage case which enables only a blade to be attached to a holder handle without coming into contact with the blade with the blade accommodated in the case, thereby providing safe attachment to the handle and enabling bacteria and the like to be prevented from adhering, has a simple structure, and can be manufactured inexpensively.

The present invention adopts following configurations to solve the above-mentioned problem. First, a holder handle already used widely is provided at its front end portion with an attaching finger in which a blade is engaged to be attached, and in the blade is formed a fit hole adapted to an engagement portion such as a slit or the like formed in the attaching finger. A storage case that accommodates the blade is comprised of a box-shaped case body having an upper support face and a lower support face to sandwich upper and lower surfaces of the plate-shaped blade therebetween to store. For example, corresponding to the shape and dimensions of the blade, the case body is formed in the shape of a box, and is provided at its inside with the upper support face and the lower support face to hold the plate-shaped blade. As the upper and lower support faces, for example, the case is divided into two parts, upper and lower cases, and the upper support face is formed in the upper case while the lower support face is formed in the lower case. Alternately, an internal lid is provided to form the upper support face therein.

In the thus formed box-shaped case body, an insertion guide hole is disposed in at least one of the upper and lower support faces, and the insertion guide hole is to guide the attaching finger along the lower surface of the internal plate-shaped blade from the outside of the case. Further, in at least one of the upper and lower support faces is disposed a blade fixing piece that engages in the plate-shaped blade to position and hold. For example, the blade fixing piece is formed of a projection such as a pin or the like that is fitted in a concave recess portion such as a notch or the like formed on the blade side, and disposed in a plurality of portions (preferably, two or three portions) to hold the blade not to displace. The blade fixing pieces are configured to separate from the plate-shaped blade by the attaching finger in an attachment state (position) where the plate-shaped blade is fitted and attached into the attaching finger entering along the insertion guide hole.

By configuring in such a manner, when the attaching finger is inserted along the insertion guide hole from the outside of the case, the engagement portion such as a slit or the like formed in the finger is fitted and attached into the fit hole of the plate-shaped blade. At this point, the plate-shaped blade is held by the blade fixing pieces and fitted and attached reliably without being pushed out by the attaching finger entering the insertion guide hole. Meanwhile, when the attaching finger is pulled out, the blade fixing pieces are released from the engagement to separate from the blade, and the attaching finger can be removed from the case with the blade attached to the attaching finger in the state where the attaching finger is attached to the case. Accordingly, injury does not occur by the blade in attaching the blade, and when the blade is sterilized in its manufacturing, bacteria and the like do not adhere to the blade during the processes of distribution and storage because the blade is protected by the case after the manufacturing.

Next, the box-shaped case body is separately formed using an upper case having the upper support face and a lower case having the lower support face, and the upper case is coupled to the lower case to be able to connect and separate to/from the lower case in a detachable or openable/closable manner. Then, the blade fixing pieces are arranged on the upper support face of the upper case to engage and hold the plate-shaped blade, and at the same time, the insertion guide hole is formed along the lower support face of the lower case. Further, the insertion guide hole is formed so that the upper case separates from the lower case in the attachment position where the inserted attaching finger is engaged in the attachment fit hole formed in the plate-shaped blade. The upper case thus separates from the lower case by the attaching finger, the blade fixing pieces are thereby released from the engagement with the plate-shaped blade, and it is possible to pull both the members from the insertion guide hole.

Next, in the case where the upper case with the above-mentioned structure separates from the lower case, it is possible to provide a configuration that the lower case and upper case are coupled to be able to connect and separate by a seizing hook having a release operating piece, and that the blade fixing pieces are released from the engagement with the plate-shaped blade by the operation of the operating piece. In this case, for example, the seizing hook and a push operating piece coupled to the hook are integrally formed in the upper case, the upper case is separated from the lower case by pressing the operating piece, and the blade fixing pieces provided in the upper case are thereby released from the engagement with the blade.

Further, in the invention, when the blade fixing pieces are configured to be able to engage and separate in/from the plate-shaped blade, it is possible to configure the pieces using a deformable elastic member to engage when the attaching finger enters while releasing the engagement when pulling out the finger, and in this case, the following configuration is adopted.

In the blade storage case to be attached detachably to the holder handle as an attachment configured as described above, the storage case is comprised of a box-shaped case body having an upper support face and a lower support face to sandwich upper and lower surfaces of the plate-shaped blade therebetween to store. In the box-shaped case body, an insertion guide hole and a blade fixing piece are disposed respectively in at least one of the upper support face and lower support face. The insertion guide hole is to guide the attaching finger along the lower surface of the plate-shaped blade from the outside of the case, while the blade fixing piece is able to engage and separate in/from the plate-shaped blade.

Then, the blade fixing piece is comprised of an elastic member that elastically engages in an engagement concave recess portion formed in the plate-shaped blade. The elastic member engages in the engagement concave recess portion when the attaching finger enters along the insertion guide hole, while releasing the engagement with the engagement concave recess portion when the finger separates. By configuring in such a manner, it is possible to attach the blade by the operation of inserting the attaching finger through the insertion guide hole, and take the blade out of the case to the outside by the operation of pulling out the finger, and the blade can be attached to the handle with a single motion.

### Advantageous Effect of the Invention

The present invention resides to a blade storage case to attach and detach a blade as an attachment to/from a holder handle provided at its front end portion with an attaching finger in which the blade is engaged to be attached, where the storage case is comprised of a box-shaped case body having an upper support face and a lower support face to sandwich upper and lower surfaces of the plate-shaped blade therebetween to store, the box-shaped case body is provided with an insertion guide hole to guide the attaching finger along the lower surface of the plate-shaped blade from the outside of the case, and with blade fixing pieces capable of engaging and separating in/from the plate-shaped blade, and the blade fixing pieces engage in the plate-shaped blade to hold when entering along the insertion guide hole, while separating from the plate-shaped blade in the attachment state where the plate-shaped blade is fitted and attached into the attaching finger, and therefore, exhibits the following effects.

First, the plate-shaped blade is attached by inserting the attaching finger from the insertion guide hole with the blade stored in the case body, the attaching finger is capable of being pulled out by pulling the finger out with the blade attached thereto, and it is thus possible to attach the blade to the handle without touching the blade. Accordingly, loading the holder handle with the blade is safe with no risk of injury, eliminates the need of touching the blade by hand and finger with no fear of bacteria adhering, and does not have the risk of resulting in infections particularly in the case of the surgical knife. Further, the storage case can be formed of an inexpensive material easy in processing such as plastic, and produces the effects of being easy in disposal processing after used concurrently with being inexpensive, and the like.

The present invention will specifically be described below based on preferred embodiments as shown in accompanying drawings.
FIG.1 contains perspective views showing appearances of a holder handle and a storage case with an internal blade when the invention is applied to a surgical knife. The perspective views show a handle body and a blade storage case according to the embodiment of invention, where FIG.1(a) shows the handle body, and FIG.1(b) shows the storage case;

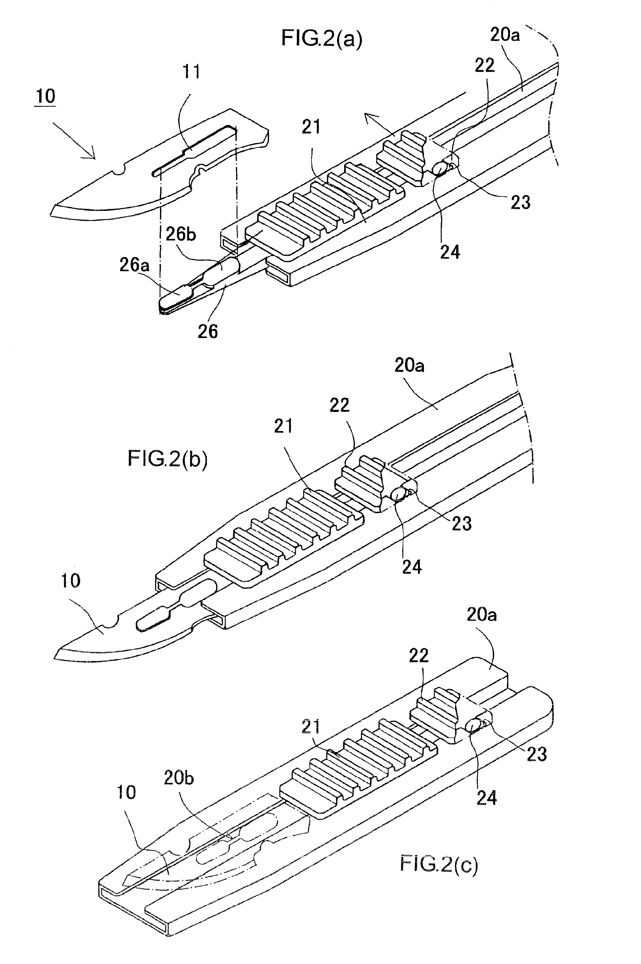

FIG.2 is an explanatory view illustrating an attachment engagement mechanism of the holder handle and the blade and shows an aspect where the blade is attached to the holder handle of FIG.1, where FIG.2(a) shows an explanatory view of an attachment state, FIG.2(b) shows an explanatory view of a usage state where the attached blade is fixed, and FIG.2(c) shows an explanatory view of a state where the used blade is stored in the holder handle;

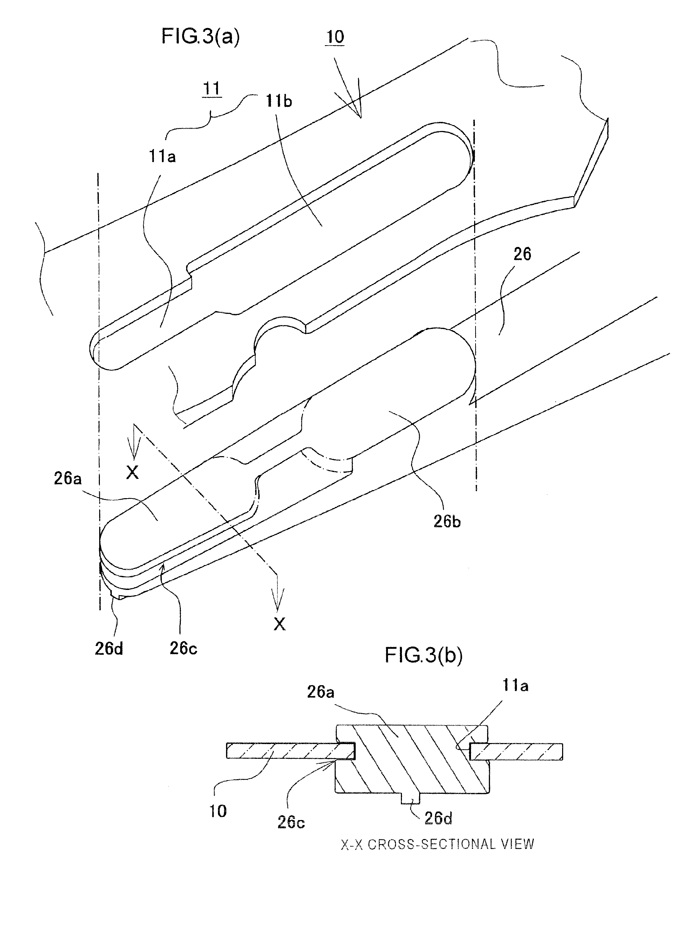

FIG.3 is another explanatory view illustrating an attachment engagement mechanism of the holder handle and the blade, and contains explanatory views of an engagement state where the blade is attached to the holder handle of FIG.1, where FIG.3(a) shows an explanatory view of the engagement relationship, and FIG.3(b) shows an X-X cross-sectional view of FIG.3(a);

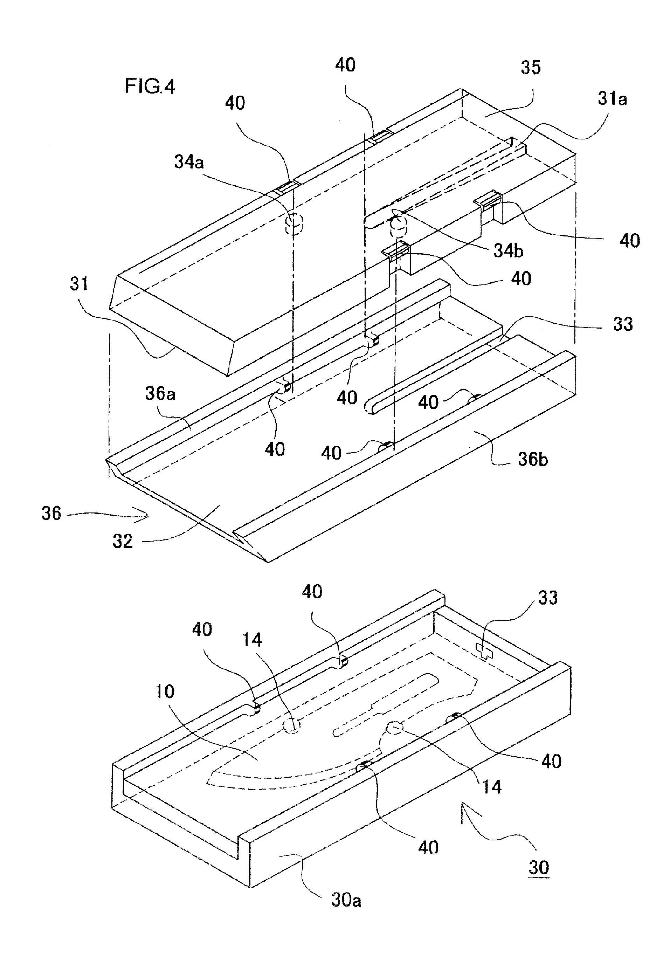

FIG.4 contains explanatory views illustrating a structure of the storage case and illustrates an exploded perspective view of the storage case of FIG.1;

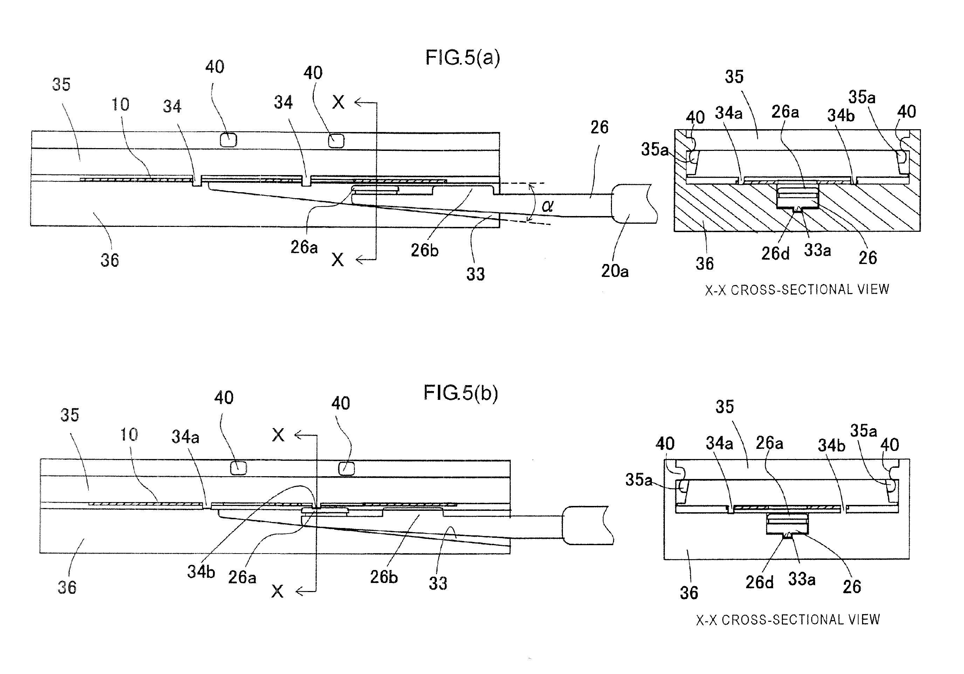

FIG.5 shows state views to attach the blade to the holder handle of FIG.1, where FIG.5 (a) shows a state view of an initial stage where the holder is inserted in the storage case, and FIG. 5 (b) shows a state view immediately before the blade is attached to the holder;

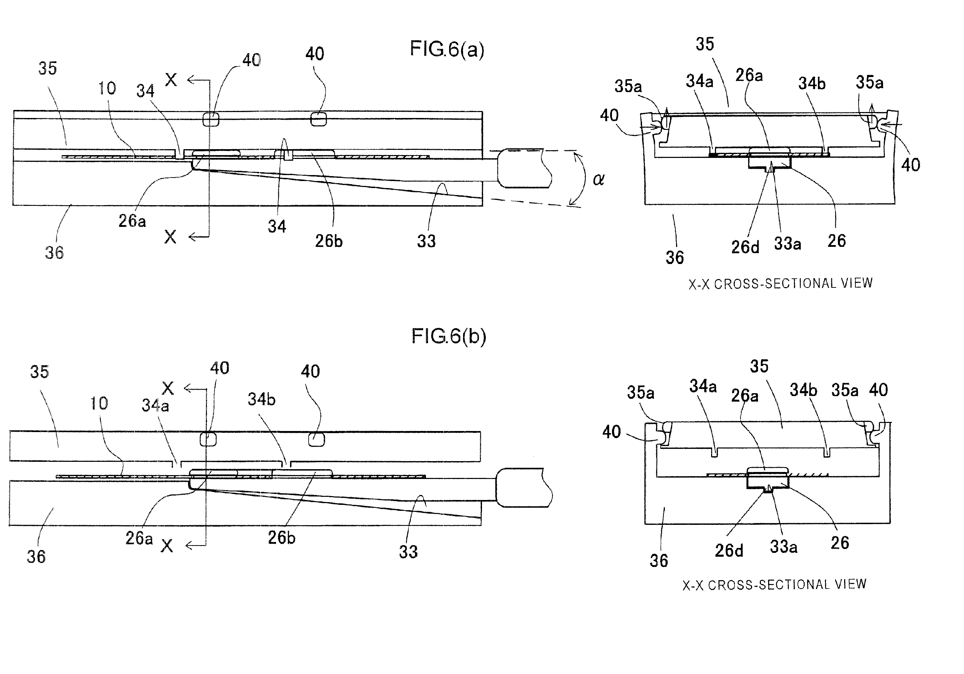

FIG.6 shows the same state view as in FIG.5, where FIG.6(a) shows a state view of a state where the blade is attached to the holder, and FIG.6(b) shows a state view of a state where the holder is pulled out of the storage case after the attachment;

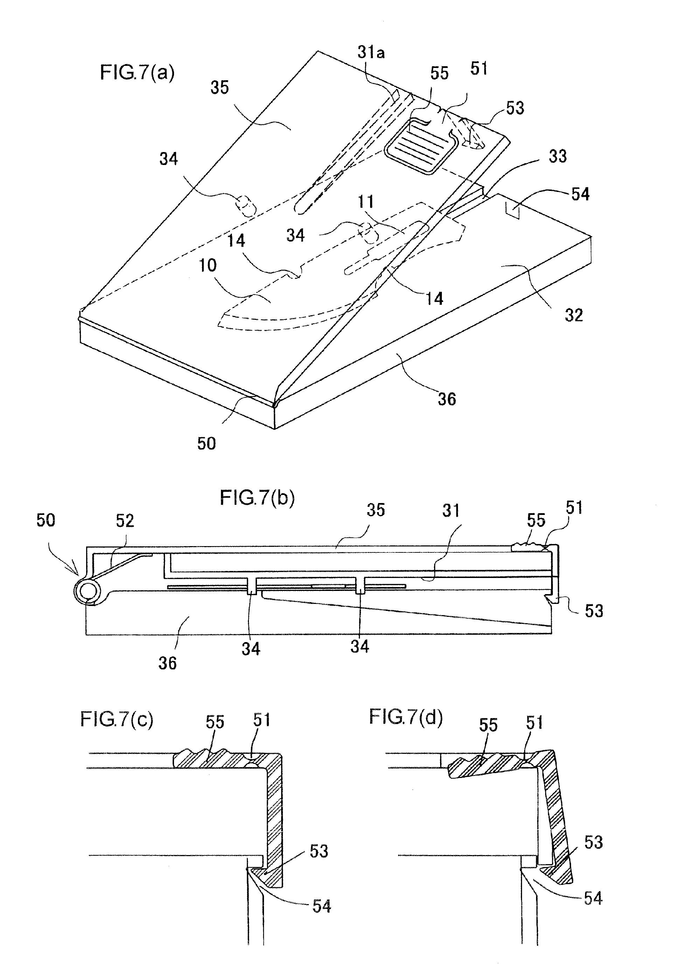

FIG. 7 shows an embodiment of the storage case different from FIG. 5, where FIG.7(a) shows a perspective view of the storage case, FIG.7(b) shows a cross-sectional view of FIG.7(a), FIG.7(c) shows a cross-sectional view of principal part, and FIG.7(d) shows a cross-sectional view of principal part in an operating state;

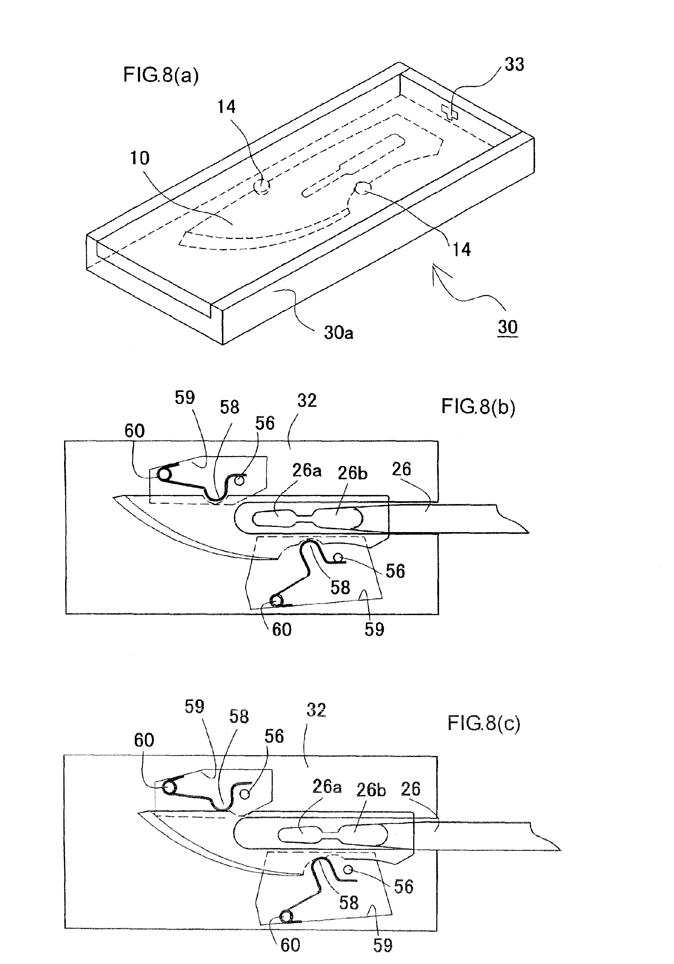

FIG.8 shows an embodiment of the storage case different from FIGs.5 and 7, where FIG.8(a) shows a perspective view of the storage case, FIG.8(b) shows a plan explanatory view of a state of inserting the holder in the case, and FIG. 8 (c) shows a state explanatory view of a state of pulling the holder out of the case; and

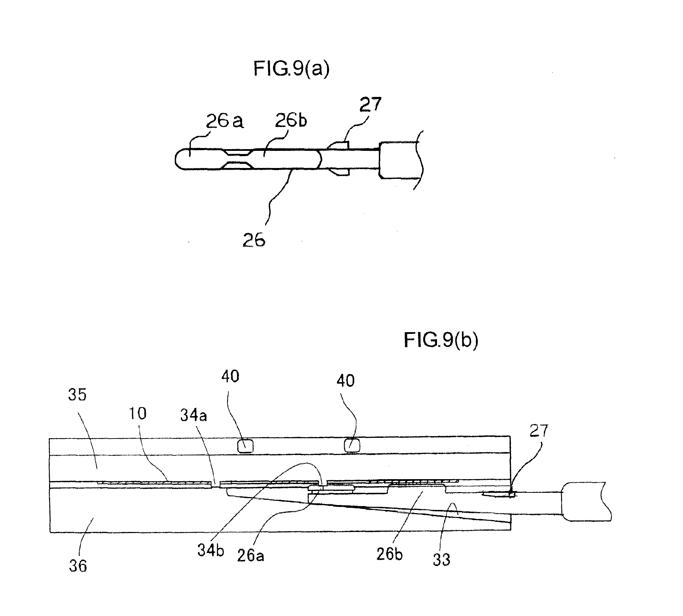

FIG.9 shows a modification of the holder handle 20 easing separation between the upper case and lower case of the storage case.

Described first is the structure of the holder handle according to the blade storage case of the invention. As shown in FIG.1, in general, a holder handle widely used for a surgical knife is comprised of a handle body 20a, and an attaching finger 26 in the shape of a bar formed to protrude from the front end of the body. The finger 26 is formed to be rectangle in cross section and in the shape of a bar with an appropriate length, and is provided with an engagement portion in the shape adapting to a fit hole 11 of the blade described later at two portions, first engagement portion 26a and second engagement portion 26b. In the first engagement portion 26a, as shown in FIG.3, a slit concave portion 26c is formed on its side face.

Meanwhile, the blade 10 is formed to have a shape of a blade edge in accordance with the intended use, and provided at its base portion with the fit hole 11 adapting to the engagement portion of the finger 26 at two portions, first fit hole 11a and second fit hole 11b. Then, the first fit hole 11a is formed in the shape of engaging in the first engagement portion 26a, while the second fit hole 11b is formed in the shape of engaging in the second engagement portion 26b. Further, in order for an edge portion of the first fit hole 11 formed in the blade to mesh with the slit concave portion 26c formed on the side face of the first engagement portion 26a, the groove thickness of the slit concave portion 26c is configured to be the shape substantially in accordance with the thickness of the blade. The blade 10 attached to the thus configured attaching finger 26 is regulated in a fit in the horizontal direction by the first and second engagement portions, while being regulated in a fit in the vertical direction by the slit concave portion 26c, and integrally attached to the attaching finger.

Further, in the holder handle as shown in the figure, the attaching finger 26 is fitted and supported slidably in a storage concave portion 20b formed in the handle body 20a, and the finger 26 is integrally provided with a slide operating piece 21 and lock operating piece 22. Then, in the storage concave portion 20b, engagement lock grooves 23 to fix the attaching finger 26 are provided in a plurality of portions, and when a lock pin (not shown) provided on the lock operating piece 22 side is engaged in the lock groove 23, the attaching finger 26 is fixed in this position. Further, when the lock operating piece 22 is moved in the direction of the arrow in FIG.2 against a biasing spring (not shown), the lock is released, and the attaching finger is movable using the slide operating piece 21. The attaching finger 26 is locked in three portions, (a) blade attachment/detachment position, (b) usage state, and (c) storage state, and particularly, in the usage state, concurrently with the blade 10 being engaged in the engagement portion of the attaching finger 26 as described previously, the base portion of the blade is fitted into the storage concave portion 20b of the handle body and is reliably fixed to the holder handle. Further, as shown in FIG. 2 (c), the blade 10 can be stored in the storage concave portion 20b of the handle body 20a.

The invention aims to enable the blade 10 to be attached to the attaching finger 26 with the blade 10 stored in the storage case 30 in attaching the blade 10 to the holder handle 20 with the above-mentioned configuration. The storage case 30 will be described below. As shown in FIG.4, the storage case 30 is formed in the shape of a box capable of storing the blade 10 corresponding to the shape of the blade 10. The storage case 30 is comprised of a box-shaped case body 30a forming a housing, an upper support face 31 and a lower support face 32 that support the blade inside the case body, an insertion guide hole 33 formed in one of the upper and lower support faces, and a blade fixing piece 34 that fixes the blade. In the case as shown in FIG. 4, the case body is comprised of an upper case 35 and a lower case 36, and the inner surface of the upper case 35 is the upper support face 31, while the inner surface of the lower case 36 is the lower support face 32. Then, the upper case 35 is detachably connected in the shape of a lid and thus coupled to the lower case 36 to be able to separate (the structure will be described below.)

Then, the lower support face 32 is provided with the insertion guide hole 33 that guides the attaching finger 26 along the lower face of the blade from the outside of the case. This is because of inserting the attaching finger 26 of the holder handle 20 as described above in the plate-shaped blade 10 stored in the case body 30a to engage the finger 26 and the blade 10 with each other, and attaching the blade to the handle. Accordingly, the insertion guide hole 33 is formed in the shape adapting to the attaching finger 26, and it is formed that the insertion of the finger engages the fit holes (the first fit hole 11a and second fit hole 11b described previously) of the blade mounted and supported on the lower support face and the first and second engagement portions 26a and 26b of the finger 26 with each other, respectively. In particular, in the case as shown in the figure, to facilitate the insertion of the finger, a guide 26d formed of a protrusion or concave portion is formed in the bottom in the longitudinal direction on the back side of the finger 26 having the engagement portions 26 on its upper face, while a guide rail 33a is formed in the insertion guide hole 33 to be fitted with the guide 26d.

By this guide rail, without exactly matching the cross-sectional shape of the insertion guide hole 33 and the cross-sectional shape of the finger, the finger is guided along the guide rail, and the insertion guide hole can thereby be manufactured readily. Further, in the insertion guide hole 33, as shown in FIG.5, the bottom is inclined an angle of α as shown in FIG.5 so that the first and second engagement portions are brought closer to the upper support face 31 side with the entry of the attaching finger. This is because the engagement portions 26a and 26b of the attaching finger slip into the fit holes 11 of the blade 10 mounted on the lower support face 32 from the insertion guide hole 33 formed in the lower support face i.e. from the state of FIG.5 (a) to the state of FIG.5(b). The insertion guide hole 33 is thus inclined. Alternately, the lower support face may be inclined to store the tilting blade, and in this case, the lower support face is inclined an angle of -α in the opposite direction.

Further, instead of forming such an incline, space may be formed between the upper and lower support faces so that the blade rises from the lower support face by the entry of the finger. In the case as shown in the figure, the insertion guide hole 33 is inclined an angle of α as shown in the figure, while a small gap is formed between the upper and lower support faces to raise the blade slightly from the lower support face. This is because the edge portion of the first fit hole 11a of the blade is caused to mesh with the slit concave portion 26c of the first engagement portion 26a of the attaching finger in the state of FIG.5.

Described herein is the coupling structure of the upper case 35 and lower case 36 as described above. As shown in FIG.4, the lower case 36 is comprised of integrally formed left and right side walls 36a and 36b, lower support face 32 and insertion guide hole 33 by synthetic resin mold forming. Further, the upper case 35 is also formed of a plate-shaped synthetic resin, the upper support face 31 is formed in the inner bottom, and the concave groove 31a is ditched in the face 31. The concave groove 31a is an escape groove to avoid causing the engagement portions 26 of the finger to interfere in pulling the attaching finger 26 inserted from the insertion guide hole 33 (formed in the lower case) as described previously out of the case with the blade attached to the finger. The upper case 35 and lower case 36 as described above are formed of a material capable of causing elastic deformation such as a synthetic resin or the like, and preferably, include an X-ray impermeable additive such as barium sulfate and the like.

Further, the upper support face 31 is provided with a blade fixing piece 34 formed of protrusions 34a and 34b obtained by planting pins. The protrusions 34a and 34b are arranged in positions that engage the protrusions in engagement concave recess portions 14 formed in the blade 10. This is because of preventing the blade from being pushed and moved in inserting the attaching finger 26 from the insertion guide hole 33, and also preventing the stored blade from rattling in the transport process. In the blade fixing piece 34 shown in the figure, in order not to cause damage to the cutting function of the blade 10, the protrusions 34a and 34b are arranged in positions to engage in the engagement concave recess portions 14 each formed of a notch on the blade side respectively in the back of the blade and in the base portion on the blade surface side. Alternately, for example, an aperture may be provided in the center of the blade so that the blade fixing piece 34 is formed using a pin adapting to the aperture. In the embodiment as shown in FIG. 4, the protrusions 34a and 34b are provided on the upper support face 31 side of the upper case 35, while the engagement concave recess portions 14 are provided on the lower case 36 side. Alternately, the engagement concave recess portions 14 may be provided on the upper support face 31 side of the upper case 35, while the protrusions 34a and 34b may be provided on the lower case 36 side.

Thus formed upper case 35 is fitted into between the left and right side walls 36a and 36b of the lower case 36 in the shape of a lid, and the left and right side walls 36a and 36b are provided with locking protrusions 40. The locking protrusions 40 are provided in a few portions as appropriate in the left and right side edges 36a and 36b so as to hold the upper case in a position that the blade 10 mounted on the lower support face 32 is held vertically between the upper support face 31 and face 32, and engage in the locking edge 35a of the upper case. In particular, the locking protrusions 40 as shown in the figure are formed to cause elastic deformation in the side walls of the lower case formed of a synthetic resin with elasticity, and thereby cause snapping operation in inserting the attaching finger 26 as shown in FIG.6(a).

In other words, when the upper case 35 is pushed upward from below, the locking edge 35a climbs over the locking protrusions 40 and separates from the lower case, and at this point, the protrusions 40 perform the snapping operation. In addition, the snapping operation can be adjusted by forming thin portions that tend to cause elastic deformation in the left and right side edges 36a and 36b, forming a slit and the like, and the protrusions as shown in the figure cause the snapping operation by thicknesses of the left and right side walls.

In the upper case 31 with the integrally formed blade fixing piece 34 as described previously, when the attaching finger 26 is inserted in the insertion guide hole 33, the front end of the first engagement portion 26a of the finger pushes the upper case 35 upward against the elastic force of the locking protrusions 40. At this point, the gap is formed between the upper support face 31 and lower support face 32, and the blade 10 is supported by the fixing piece 34, and is only movable vertically. In this state, the edge portion of the first fit hole 11 formed in the blade is fitted into the slit concave portion 26a of the attaching finger 26. In the state of FIG.6(a) where the fit is completed, the first engagement portion 26a of the attaching finger 26 pushes the upper case 35 upward by the upper face thereof, the locking edge 35a climbs over the locking protrusions 40, and the upper case 35 separates from the lower case 36. After the separation, the upper case 35 is held by elastic recovery of the locking protrusions 40 while floating on the protrusions 40, and the blade fixing piece 34 of the upper case separates from the fit hole 11 of the blade. At the same time, a blade pull-out opening 33 is formed between the upper case 35 and lower case 36.

Referring to FIGs.5 and 6, described next is a state where the blade 10 stored in the storage case 30 as described above is attached to the attaching finger 26 of to the holder handle 20. FIG.5(a) shows a state where the blade 10 is stored in the storage case 30 as shown in FIG.4, and the blade 10 is sandwiched between the upper support face 31 and lower support face 32 and supported by the blade fixing piece 34. The case is distributed in this state, and does not rattle during the transport process. In addition, the insertion guide hole 33 is opened, but is preferably sealed by a seal or packing during the distribution process.

FIG.5 (a) shows an initial state of the attaching finger 26 of the holder handle 20 being inserted into the insertion guide hole 33 after removing the packing. At this point, the guide 26d formed of the protrusion on the attaching finger 26 side engages in the guide rail 33a formed in the insertion guide hole and smoothly enters. In this state, the blade 10 is supported by the blade fixing pieces 34 (two portions in the figure) protruding from the upper support face 31 being fitted into the engagement concave recess portions 14.

Next, FIG.5(b) shows a state where the attaching finger 26 further travels forward in the insertion guide hole 33 and comes into contact with the lower face of the blade 10. As described previously, since the insertion guide hole 33 is inclined an angle of α as shown in the figure, the first engagement portion 26a located at the front end of the attaching finger 26 comes into contact at its upper face with the lower face of the blade, and pushes the blade upward. At this point, the blade 10 is supported by the engagement concave recess portions 14 engaging in the blade fixing pieces 34, and floats along the pins forming the fixing pieces without moving horizontally. Next, when the attaching finger 26 travels to the position of the attachment fit hole 11 of the blade 10, the first engagement portion 26a at the front end of the finger engages in the first fit hole 11a of the blade 10. This engagement is made by the slit concave portion 26c on the side face of the first engagement portion 26a meshing with the edge portion of the fit hole 11a.

When the attaching finger 26 further travels forward in this engagement state, the finger 26 reaches the attachment position of FIG.6(a), and the first engagement portion 26a on the finger side is fitted into the first fit hole 11a on the blade side, while the second engagement portion 26b is fitted into the second fit hole 11b. Concurrently with the fit, the upper face of the first engagement portion 26a pushes the upper support face 31 upward. A push-up amount is set to cause an engagement state such that the blade fixing piece 34 does not separate from the engagement concave recess portion 14 by the inclined angle of α of the attachment fit hole.

Meanwhile, in the upper case 35 and lower case 36, the locking protrusions 40 and locking edge 35a are fitted with each other as described previously, and in the states of FIGs.5(a) and 5(b), the upper case is held in the position that the upper support face 31 supports the blade 10. When the upper case 35 is pushed up by the attaching finger 26, as shown in FIG.6 (a), the lower case 36 bends and is deformed. In this state, the blade fixing piece 34 is engaged with the blade 10. In the state as shown in FIG.6(a), the blade 10 is completely attached to the attaching finger 26, and is fixed to the finger. When the attaching of the blade is completed, the locking protrusions 40 of the lower case 36 recover to the original state from the state in FIG.6(a) where the protrusions 40 are elastically deformed after a time delay.

FIG.6(b) shows the recovery state, where the locking protrusions 40 on the lower case 36 side push the locking edge (protrusion) 35a on the upper case 35 side upward. Then, the upper case 35 separates from the lower case 36, and the blade fixing piece 34 formed in the upper support face 31 is removed from the engagement concave recess portion 14 of the blade 10, and releases the engagement. Therefore, when the attaching finger 26 of the holder handle 20 is pulled out of the insertion guide hole 33, the blade 10 attached already is pulled out of the storage case integrally with the finger. In this state, the blade attached to the holder handle 20 is used as a normal blade in the usage state of FIG.2 (b) and in the storage state of FIG.2(c).

Next, the embodiment as shown in FIG.7 intends to release the blade fixing piece 34 from the blade 10 by an operating piece 55 provided in the case housing after attaching the blade to the attaching finger 26 in the state where the blade fixing piece is engaged in the blade inside the storage case. The other components are the same as in FIG.5, and assigned the same reference numerals to omit descriptions thereof. In the same way as described above, the lower case 36 is provided with the lower support face 32 and insertion guide hole 33. The lower case 36 is provided with the integrally-formed upper case 35 to be openable and closable by a hinge portion 50. As the hinge portion 50, the upper case and lower case are integrally formed with a synthetic resin high in bending strength, and the hinge portion 50 is formed of a bendable thin portion in the formation. The hinge portion is provided with a spring 52 integrally formed in resin molding so as to open (upward as viewed in FIG. 7) the upper case 35 around the hinge portion 50.

Then, the upper case 35 is provided with an integrally-formed lock hook 53 on the side edge on the side opposite to the hinge portion 50, and the lower case 36 is provided with a concave recess hook 54 in which the lock hook 53 engages. Then, the operating piece 55 is integrally formed with the upper case 35 to enable the piece 55 to be pressed down, elastically deforms from the state of FIG.7(b) to the state of FIG.7(C) around a bending portion 51, and is configured to separate the lock hook 53 from the concave recess hook 54 of the lower case. The thus configured upper case 35 is provided with the upper support face 31 and blade fixing piece 34 formed of a pin in the same way as in the case described previously. Accordingly, when the operating piece 55 is operated to release the lock hook 53 from the concave recess hook 54, the upper case 35 turns upward around the hinge portion 50 by the spring 52, and the blade fixing piece 34 provided in the upper case 35 separates from the blade 10.

Then, as in the case shown in FIG. 5, the attaching finger 26 of the holder handle 20 is inserted in the insertion guide hole 33 formed in the lower case 36, and the first and second engagement portions 26a and 26b on the finger side are respectively fitted into the first fit hole 11a and second fit hole 11b on the blade side. After completing the attachment of the blade 10, an operator presses the operating piece 55 downward, and releases the upper case 35 upward. After confirming that the blade 10 is attached, when the operator pulls the attaching finger 26 out of the insertion guide hole 33, the blade 10 attached already is pulled out of the storage case integrally with the finger. In this state, the blade attached to the holder handle 20 is used as a normal blade in the usage state of FIG.2(b) and in the storage state of FIG.2(c).

Next, in a case as shown in FIG.8, a blade fixing piece as described previously is formed of an elastic member, the fixing piece 60 engages in the concave recess portion 14 of the blade 10 to inhibit the blade from moving when the attaching finger 26 enters the insertion guide hole 33, while being released from the engagement with the concave recess portion 14 when pulling out the attaching finger 26, and it is thus intended to enable the finger to be pulled out with the blade attached thereto. The other components are the same as in FIG. 5 and assigned the same reference numerals to omit descriptions thereof.

As in the case described previously, the blade 10 is mounted and held on the lower support face 32, and the engagement concave recess portion 14 is beforehand formed in the blade. Then, the lower support face is provided with a step-height concave recess portion 59, the concave recess portion supports a blade fixing piece 58 formed of a spring member by a fixing pin 60, and the front end portion of the blade fixing piece 58 is positioned to engage in the engagement concave recess portion 14 of the blade. Then, the base portion of the fixing piece 58 is locked by a lock wall 59a of the height-step concave recess portion 59 and always biased toward the engagement concave recess portion 14 side. "56" in the figure denotes a stopper pin, and acts to inhibit the movement when the blade 10 is pushed into the left side as viewed in the figure.

Then, as in the case shown in FIG. 5, the attaching finger 26 of the holder handle 20 is inserted in the insertion guide hole 33 formed in the lower case 36, and the first and second engagement portions 26a and 26b on the finger side are respectively fitted into the first fit hole 11a and second fit hole 11b on the blade side. At this point, although the blade 10 is pushed into the left side as viewed in the figure by the attaching finger 26, the blade fixing piece 58 is inhibited from turning by the stopper pin 56, and holds the blade in the predetermined position. Then, after completing the attachment of the blade 10, when the operator pulls the attaching finger 26 out of the insertion guide hole 33, the blade fixing piece 58 turns around the fixing pin 60 as shown in FIG.8(c), and the blade 10 is pulled out of the storage case integrally with the finger. In this state, the blade attached to the holder handle 20 is used as a normal blade in the usage state of FIG.2(b) and in the storage state of FIG.2(c).

FIG.9 shows a modification of the holder handle 20. As shown in FIG. 9 (a), a wing member 27 may be provided on the back side of the attaching finger 26 of the holder handle 20 to easily separate the upper case 35 and lower case 36 constituting the storage case. As shown in FIG.9(b), in inserting the attaching finger 26 of the holder handle toward the back side of the insertion guide hole 33 of the storage case to separate the upper case 35 and lower case 36, the wing member 27 enters the gap between the upper case 35 and lower case 36, and eases the separation between the upper case 35 and lower case 36.

The present invention relates to a storage case that accommodates a blade of a surgical knife and the like, more particularly to a storage case of a blade to attach and detach the blade as an attachment to/from a front end of a handle portion of a holder or the like, and has industrial applicability.

## Claims

1. A blade storage case of a knife or the like attached detachably as an attachment to an attaching finger extending from a front end portion of a holder handle to engage in the blade to attach the blade, comprising:
a box-shaped case body having an upper support face and a lower support face to sandwich upper and lower surfaces of the plate-shaped blade therebetween to store,
the box-shaped case body further having:
an insertion guide hole disposed in at least one of the upper support face and the lower support face, the insertion guide hole guiding the attaching finger along a lower surface of the plate-shaped blade from the outside of the case; and
a blade fixing piece disposed in at least one of the upper support face and the lower support face, the blade fixing piece capable of engaging and separating in/from the plate-shaped blade,
wherein the blade fixing piece separates from the plate-shaped blade in an attachment state where the plate-shaped blade is engaged and held by the attaching finger entering along the insertion guide hole.

2. The blade storage case according to claim 1, wherein the box-shaped case body is separately formed using an upper case having the upper support face and a lower case having the lower support face,
the upper case is coupled to the lower case to be able to connect and separate to/from the lower case in a detachable or openable/closable manner,
the blade fixing piece is disposed on the lower support face of the lower case to engage and hold the plate-shaped blade,
the insertion guide hole is formed along the lower support face of the lower case,
the insertion guide hole is formed so that the upper case separates from the lower case in an attachment position where the inserted attaching finger is engaged in an attachment fit hole formed in the plate-shaped blade, and
the blade fixing piece is released from the engagement with the plate-shaped blade by the upper case separating.

3. The blade storage case according to claim 2, wherein in the insertion guide hole and the lower support face mounting and supporting the plate-shaped blade, a bottom of the insertion guide hole and/or the lower support face is inclined so that an upper surface of the attaching finger gradually approaches to the upper support face side as the attaching finger enters.

4. The blade storage case according to claim 2, wherein the blade fixing piece formed in the lower case is formed of a protrusion such as a pin engaging in an engagement concave recess portion formed in the plate-shaped blade,
the upper case and lower case are connected using an elastically deformable engagement/separation mechanism while being fitted in the mechanism, and
the engagement/separation mechanism holds the lower case in a connection position where the protrusion engages in the plate-shaped blade, and in a separation position where the protrusion separates from the plate-shaped blade.

5. The blade storage case according to claim 4, wherein at least one of the upper case and the lower case is formed of an elastically deformable material such as a synthetic resin or the like,
the engagement/separation mechanism is formed of a protrusion or a concave portion formed on the lower case and the upper case to be able to engage and separate in/from each other, and
the protrusion or the concave portion moves the lower case from the connection position to the separation position to hold by elastic deformation of the case.

6. The blade storage case according to claim 1, wherein the box-shaped case body is separately formed using an upper case having the upper support face and a lower case having the lower support face,
the upper case is coupled to the lower case to be able to connect and separate to/from the lower case using a lock hook having a release operating piece in a detachable or openable/closable manner,
the blade fixing piece is disposed on the upper support face of the upper case to engage and hold the plate-shaped blade, and
the blade fixing piece is released from the engagement with the plate-shaped blade when the upper case separates from the lower case by the operating piece.

7. A blade storage case of a knife or the like attached detachably as an attachment to an attaching finger extending from a front end portion of a holder handle to engage in the blade to attach the blade, comprising:
a box-shaped case body having an upper support face and a lower support face to sandwich upper and lower surfaces of the plate-shaped blade therebetween to store,
the box-shaped case body further having:
an insertion guide hole disposed in at least one of the upper support face and the lower support face, the insertion guide hole guiding the attaching finger along a lower surface of the plate-shaped blade from the outside of the case; and
a blade fixing piece disposed in at least one of the upper support face and the lower support face, the blade fixing piece capable of engaging and separating in/from the plate-shaped blade,
wherein the blade fixing piece is formed of an elastic member to elastically engage in an engagement concave recess portion formed in the plate-shaped blade, and the elastic member engages in the engagement concave recess portion when the attaching finger enters along the insertion guide hole, while being elastically deformed to release the engagement with the engagement concave recess portion when the attaching finger separates.

8. The blade storage case according to claim 1 or 7, wherein a guide rail is formed in the insertion guide hole, the guide rail adapting to a guide formed of a protrusion or a concave portion formed in the attaching finger.
